# EUROPEAN PATENT APPLICATION

(11) **EP 0 578 356 A2**
(43) Date of publication of application: **12.01.1994**
(21) Application number: 93303758.2
(22) Date of filing: 14.05.1993
(51) Int. Cl.: C07K 15/00, C12P 21/08, G01N 33/577, C12N 15/31

(54) **Mycoplasma protein promoting metastases and antibody against it**

(30) Priority: 14.05.1992 JP 166659/92
(71) Applicant: KABUSHIKI KAISHA HAYASHIBARA SEIBUTSU KAGAKU KENKYUJO, Okayama-shi Okayama (JP)
(72) Inventor: Iwaki, Kanso, Okayama-shi, Okayama (JP); Ushio, Shimpei, Okayama-shi, Okayama (JP); Kurimoto, Masashi, Okayama-shi, Okayama (JP)
(74) Representative: Daniels, Jeffrey Nicholas

(57) **Abstract**

Disclosed are a novel protein having an activity to promote metastases of cancers, and its antibody and preparation. The novel protein is prepared from a microorganism of the genus Mycoplasma, and significantly used in studies on cancer metastases. The antibody recognizes the novel protein so that it can be advantageously used as a diagnostic agent for cancer metastases, as well as a cancer metastasis-inhibitory agent.

## Description

The present invention relates to a novel protein, and its antibody and preparation, more particularly, to a novel protein with a cancer metastasis-promoting activity, and its antibody and preparation.

Surgical operations, chemotherapies and radio-therapies are mainly used to treat cancers. It has been noted that the above-mentioned treatments usually lead to successful results but may spread in patient's body a part of cancer cells which still remain after such a treatment, and this may cause serious metastases of cancers and shorten the patient's life-span.

It is said that if metastases of cancers are overcome pains of cancer patients would be relieved and their life-spans would be much more extended. Because of this, advances in studies on metastases of cancers have been in great demand. The metastases of cancers are, however, deemed to occur or to be induced through complicated steps of (1) release of metastatic cells from original affected sites in patients, (2) invasion of the cells into patients' veins, (3) adhesion of the cells to patients' intravascular endotheliocytes, (4) subsequent invasion into patients' organs, and (5) proliferation therein, and these complicate studies on metastases of cancers. The present inventors have engaged long in such a study, and found that there exists an antigen, which relates to metastases of cancers, on the surface of RPMI 4788 cell (FERM BP-2429), an established cell line derived from human colon cancer, as well as presenting this at the meeting of the American Association for Cancer Research, the abstract of which was published in Proceedings of 81^{st}Annual Meeting of the American Association for Cancer Research, Vol.31, page 298 (1990). While C. Schmidhauser et al. reported in Journal of Cell Science, Vol.95, pp.499-506 (1990) that a microorganism of the genus Mycoplasma augments the invasiveness of cancer cells but not characterized the relevant substance nor revealed the mechanism of such an invasion. Thus, complete elucidation thereof is in great demand.

As described above, advances in studies on cancer metastases are in great demand.

The present inventors found a novel substance with a cancer metastasis-promoting activity, as well as developing its antibody and preparation; thus, the present invention provides a useful means to reveal the mechanism of cancer metastases. The present novel substance, i.e. a proteinaceous substance, as well as its antibody which recognizes a partial amino acid sequence on the protein, can be used as a diagnostic agent for metastasis of cancers, as well as a cancer metastasis-inhibitory agent.

To obtain a novel substance which relates to cancer metastases, the present inventors have studied on such a substance with RPMI 4788 cells (FERM BP-2429) having a metastatic activity, an established cell line derived from human colon cancer, said cells having been allowed to infect with a microorganism of the genus Mycoplasma prior to use.

As a result, the present inventors prepared a monoclonal antibody by immunizing animals with the cells as an antigen, and recovering the resultant monoclonal antibody. The present inventors first prepared a cancer metastasis-promoting substance by solubilizing the above-mentioned cells, and purifying and recovering the resultant solubilized material on an affinity chromatography using a gel immobilized with the monoclonal antibody, then studied the physicochemical properties of the substance. The present inventors revealed that the substance is a novel protein with a cancer metastasis-promoting activity, and established the preparation thereof and a monoclonal antibody which recognizes the novel substance. Thus, the present inventors accomplished this invention. The novel protein according to this invention possesses the following physicochemical properties:
(1) Molecular weight
   45,000±5,000;
(2) Isoelectric point
   pI = 6.0±1.0;
(3) Partial amino acid sequence
   Possessing partial amino acid sequences of Glu-Thr-Asp-Lys-Glu-Gly and Phe-Pro-Asn-Asp-Glu-Ala-Lys;
(4) Ultraviolet absorption spectrum
   Exhibiting a maximum absorption spectrum around a wave length of 280nm;
(5) Solubility in solvent
   Soluble in water, physiological saline and phosphate buffer;
(6) Cancer metastasis-promoting activity
   Possessing an activity to promote the metastasis of RPMI 4788 cell (FERM BP-2429) derived from a human colon cancer; and
(7) Stability of cancer metastasis-promoting activity
   Inactivated in an aqueous solution (pH 7.2) under incubating conditions of 100°C for 10 minutes.
   Stable in an aqueous solution (pH 7.2) under storage conditions of 4°C for one month.

### Detailed Description of the Invention

The present invention relates to a novel protein, and its antibody and preparation, more particularly, to a novel protein with a cancer metastasis-promoting activity, and its antibody and preparation.

The following Experiments will explain the present invention more in detail.

### Experiment 1

### Preparation of antibody directed against cancer metastasis-promoting substance

A seed culture of RPMI 4788 cell (FERM BP-2429) was cultured in usual manner in a nutrient culture medium, and about 2x10⁷ cells of the proliferated cells were added with the microorganism of the species Mycoplasma arginini (hereinafter referred to as "M.arginini") in an amount of about 1x10⁵ colony-forming units (CFU). The infected RPMI 4788 cells, which have a relatively-high metastatic activity per se, were allowed to proliferate in vitro in usual manner, and about 3X10⁶ cells of the RPMI 4788 cells were intraperitoneally injected to mice 4 times in total at intervals of 2 weeks to effect immunization. The spleens of the mice were extracted, cut into pieces and disaggregated in a nutrient culture medium to form a cell suspension, after which the spleen cells and P₃-X63-Ag8 cells, a mouse myeloma cell commercialized by Flow Laboratories, McLean, Virginia, USA, about 10⁴ cells each, were cosuspended in a serum-free Eagle's minimum essential medium but containing 50% polyethylene glycol-1000, followed by the incubation at 37°C for 5 minutes. The culture thus obtained was diluted 20 times in a fresh preparation of the same nutrient culture medium, and the resultant hybrid cells capable of proliferating in a nutrient culture medium added with hypoxanthine-aminopterine-thymidine were selected in accordance with the method of R. L. Davidson and P. S. Gerald described in Somatic Cell Genetics, Vol.2, No.2, pp.165-176 (1976), followed by the isolation of a hybrid cell clone capable of producing an antibody which was highly specific to RPMI 4788 cells as antigen. About 10⁶ cells of the specific hybrid cell clone were intraperitoneally transplanted to mice which were then bred for about 2 weeks. Thereafter, their abdominal dropsies and bloods were collected and centrifuged to obtain a supernatant, which was then subjected to an affinity chromatography using "Protein G Sepharose Column", a column product of Pharmacia LKB Biotechnology AB, Uppsala, Sweden, to obtain a purified monoclonal antibody. The monoclonal antibody showed a relatively strong inhibition on the metastasis-inhibitory activity of RPMI 4788 cells which had been infected with the microorganism of the genus Mycoplasma.

### Experiment 2

### Preparation and physicochemical properties of protein with cancer metastasis-promoting activity

A seed culture of RPMI 4788 cells, which had been infected with a microorganism of the genus Mycoplasma, was allowed to proliferate in usual manner in culture flasks containing a nutrient culture medium, and the proliferated cells were physically detached from the inside walls of the culture flasks by using a rubber policeman or a cell scraper, and centrifugally washed. The resultant cells were added with octylglucoside, commercialized by Dojindo Laboratories, Kumamoto, Japan, to give a concentration of 80mM, and solubilized by an overnight standing at 4°C, after which the resultant was filtered and subjected to an affinity chromatography using a gel immobilized with a monoclonal antibody prepared by the method in Experiment 1 to obtain a protein with a cancer metastasis-promoting activity. The purified protein was recovered in the yield of about one mg per 30L of the culture.

The physicochemical properties of the present novel protein were studied by using the purified protein.
(1) Molecular weight
   In accordance with the method of U. K. Laemmli described in Nature, Vol.227, pp.680-685 (1970), the purified protein was subjected to SDS-PAGE (sodium dodecylsulfate-polyacylamide gel electro-phoresis), followed by determining the molecular weight based on relative mobilities of the purified protein and marker proteins. As a result, the molecular weight of the purified protein was 45,000±5,000;
(2) Isoelectric point
   The isoelectric point of the purified protein was in the range of 6.0±1.0 when analyzed on "AMPHOLINE PAGPLATE" (pH 3.5-9.5), a gel for isoelectric point electrophoresis commercialized by Pharmacia LKB Biotechnology AB, Uppsala, Sweden;
(3) Partial amino acid sequence
   The purified protein was first digested by "TPCK-trypsin", a product of Sigma Chemical Company, ST. Louis, USA, then subjected to a reverse-phase chromatography using "Hi-Pore RP-318 column", a column product of Bio-Rad Laboratories, New York, USA, followed by recovering peptide fragments. Thereafter, two of the fragments were analyzed on "470A Protein Sequencer", an amino acid sequencer commercialized by Applied Biosystems, Inc., Foster City, USA. As a result, it was found that the purified protein has the partial amino acid sequences of Glu-Thr-Asp-Lys-Glu-Gly and Phe-Pro-Asn-Asp-Glu-Ala-Lys;
(4) Ultraviolet absorption spectrum
   The UV spectrum of the purified protein had a maximum absorption at a wave length around 280nm when analyzed on "UV-Visible Recording Spectrophotometer UV250", a spectrophotometer commercialized by Shimadzu Corporation, Kyoto, Japan;
(5) Solubility in solvent
   The purified protein was soluble in water, physiological saline and phosphate buffer;
(6) Cancer metastasis-promoting activity
   In accordance with the method of Y. Naomoto et al. described in Journal of Cancer Research Clinical Oncology, Vol.113, pp.544-549 (1987), the cancer metastasis-promoting activity of the purified protein was assayed with a model of metastasis wherein RPMI 4788 cells uninfected with Mycoplasma are used to induce a lung-cancer metastasis in nude mice. In a test group consisting of 5 BALB/c nude mice, 6-8 weeks old and about 20g weight each, 0.2ml aliquots of a phosphate buffer containing the purified protein were intravenously injected twice to the mice from their tails 24- and 3-hours before the transplantation of the RPMI 4788 cells. Thereafter, the mice were similarly injected with 2x10⁶ cells of the RPMI 4788 cells which had been suspended in a 0.2ml phosphate buffer containing the purified protein. Twenty-four hours after the transplantation of RPMI 4788 cells, the mice were similarly injected once more with a 0.2ml phosphate buffer containing the purified protein. In a control group, mice were similarly treated as above except for the use of phosphate buffer free from the purified protein. On 21st day after the transplantation of the RPMI 4788 cells, the mice were anatomized, and the metastasized lesions observed on the surface of lungs were macroscopically counted. The metastasis-promoting activity was judged as positive when the following conditions were fulfilled: (i) The average number of metastasized lesions observed in the mice of the test group was 50 or more; (ii) the average number of the metastasized lesions in the mice of the test group was augmented by 2-fold higher than that of the control group; and (iii) the augmented number is statistically significant. The metastasis-promoting activity of the purified protein was studied with a phosphate buffer containing 150µg/ml of the purified protein in accordance with the above-mentioned method. As a result, the metastasized lesions in the control group (5 nude mice) was 100±53, while in the test group administered with the purified protein 1,000 or more metastasized lesions were observed in all mice and an obvious metastasis-promoting activity was found. The metastasis-promoting activity was statistically significant. A purified monoclonal antibody prepared by the method in Experiment 1 was added to a phosphate buffer containing 150µg/ml of the purified protein to give a concentration of 1,250µg/ml, and the resultant solution was allowed to stand at an ambient temperature for 2 hours so that the purified protein was specifically bound to the monoclonal antibody. The resultant purified protein which had been treated with the monoclonal antibody was assayed for its metastasis-promoting activity similarly as above. As a result, the metastasized lesions formed in 5 mice in a test group treated with the protein, which had been neutralized with the monoclonal antibody, were 335±139, while those in 5 mice in a control group with a phosphate buffer containing only 1,250µg/ml of the same fresh preparation of the purified monoclonal antibody were 230±64. No statistically significant difference was observed in the 2 groups; and
(7) Stability of cancer metastasis-promoting activity
   No activity of a cancer metastasis-promoting activity was detected when the purified protein was assayed on the above-mentioned method after incubated at pH 7.2 and 100°C for 10 minutes. No activity loss was found when the purified protein was assayed similarly as above after stored at pH 7.2 and 4°C for one month.

### Experiment 3

### Identification of the origin of protein with cancer metastasis-promoting activity

The methods used in the following experiments were usual methods in the art which are detailed in experimental manuals, for example, T. Maniatis et al., Molecular Cloning, A Laboratory Manual, 2nd Edition, published by Cold Spring Harbor Laboratory Press, New York, USA (1989) and M. Muramatsu, Labomanual Genetic Engineering, published by Maruzen Co., Ltd., Tokyo, Japan (1988).

### Experiment 3(1)

### Preparation of radiolabeled DNA probe

Among the partial amino acid sequences obtained in Experiment 2(3), the amino acid sequence of Glu-Thr-Asp-Lys-Glu-Gly was chosen, and a nucleotide sequence estimated therefrom was synthesized using a DNA synthesizer commercialized by Applied Biosystems, Inc., Foster City, USA. The nucleotide sequence was radiolabeled in usual manner with [γ-³²P] ATP and T4 polynucleotide kinase, and the resultant 64 nucleotide sequences were tabulated in Table 1.

**Table 1**

| | | | | | |
|---|---|---|---|---|---|
| GAG | ACG | GAT | AAG | GAG | GG |
| A | A | C | A | A | |
| | T | | | | |
| | C | | | | |

These nucleotide sequences were used in the following Experiment 3(3) as radiolabeled DNA probe.

### Experiment 3(2)

### Preparation of chromosomal DNA from microorganism of the species Mycoplasma arginini

A seed culture of Mycoplasma arginini (hereinafter abbreviated as "M. arginini") IFO 14476 was inoculated to one liter of a nutrient culture medium containing a prescribed amount of "PPLO broth w/o CV", a medium of Difco Laboratories, Detroit Michigan, USA, supplemented with 15 v/v % of a horse serum commercialized by Flow Laboratories, McLean, Virginia, USA, and 0.05 w/v % of a yeast extract commercialized by Difco Laboratories, Detroit, Michigan, USA, and cultured at 37°C for 3 weeks under stationary conditions. After completion of the culture, the resultant culture was subjected to centrifugation, followed by collecting M. arginini cells and isolating the DNA in usual manner.

### Experiment 3(3)

### Southern hybridization with radiolabeled DNA probe

The DNA of M. arginini in the Experiment 3(2) was digested by a restriction enzyme of EcoRI commercialized by Takara Shuzo Co., Ltd., Kyoto, Japan, and the resultant was subjected to an agarose electrophoresis, followed by isolating DNA fragments. The DNA fragments were transferred on a nitrocellulose membrane by the blotting technique. The DNA fragments on the membrane were prehybridized with salmon sperm DNA commercialized by Sigma Chemical Company, St. Lous, USA, in order to prevent possible non-specific bindings, and the prehybridized DNA fragments were subjected to the southern hybridization with the radiolabeled DNA probe in Experiment 3(1). The nitrocellulose membrane was then placed on an X-ray film, and the resultant was subjected to a radioautography, followed by the check of the coupling of the chromosomal DNA of M. arginini and the radiolabeled DNA probe in order to identify the presence of a DNA sequence coding the protein with a cancer metastasis-promoting activity in the chromosome of M. arginini. As a result, a distinct band, which corresponds to the coupling of the chromosomal DNA of M. arginini and the radiolabeled DNA probe, was found, and because of this the present novel protein with a cancer metastasis-promoting activity is derived from M. arginini.

The following examples are the preferred embodiments according to the present invention.

### Example 1

### Preparation of novel protein from M. arginini

By using the method in Experiment 3(2), a seed culture of M. arginini IFO 14476 was cultured, and the resultant culture was centrifuged to collect M. arginini cells. The cells thus obtained were ultrasonically disrupted while cooling, and the resultant was filtered, then subjected to an affinity chromatography using a gel immobilized with a monoclonal antibody which recognizes a protein with a cancer metastasis-promoting activity, followed by recovering a protein with a cancer metastasis-promoting activity. The yield of the protein was about 0.2mg, on a dry solid basis (abbreviated as "d.s.b." hereinafter), per litter of the resultant culture. The protein had the same physicochemical properties as the novel protein with a cancer metastasis-promoting activity in Experiment 2 prepared from RPMI 4788 cells which had been infected with a microorganism of the genus Mycoplasma.

### Example 2

### Preparation of novel protein from LoVo cells infected with M. arginini

LoVo cells (ATCC CCL 229), an established cell line derived from human colon cancer, were infected with M. arginini IFO 14476, and the resultant LoVo cells were then allowed to proliferate in usual manner in culture flasks containing a nutrient culture medium. In accordance with the method in Experiment 2, the resultant cells were solubilized and filtered, and the resultant filtrate was subjected to an affinity chromatography using a gel immobilized with a monoclonal antibody which recognizes a protein with a cancer metastasis-promoting activity to obtain a protein with a cancer metastasis-promoting activity. The yield of the protein was about 2mg, d.s.b., per 30L of the resultant culture.

The product had the same physicochemical properties as the novel protein with a cancer metastasis-promoting activity in Experiment 2 prepared from RPMI 4788 cells which had been infected with a microorganism of the genus Mycoplasma.

### Example 3

### Preparation of monoclonal antibody directed against protein with cancer metastasis-promoting activity

Mice were immunized 4 times in total at intervals of 2 weeks with successive intraperitoneal injections of 0.1mg per shot of a protein with a cancer metastasis-promoting activity prepared by the method in Experiment 2. The mice were then anatomized, and the spleens of the mice were extracted and cut into pieces which were then fused in accordance with the method in Experiment 1 with Sp2/0-Ag 14 cells, a mouse myeloma cell commercialized by Dainippon Pharmaceutical Co. , Ltd. , Osaka, Japan. Among the resultant hybrid cells, hybrid cell clone, which specifically binds to the protein used as antigen and produces an antibody having a specific binding activity to the protein, was prepared. It was found that the selected hybrid cell clone produces per one ml of the resultant culture a monoclonal antibody capable of specifically binding to 10µg, d.s.b., of a protein with a cancer metastasis-promoting activity. The isotype of the monoclonal antibody was IgG2a.

The monoclonal antibody can be used as an antibody for a preparation of gel wherein a monoclonal antibody is immobilized, said antibody being used in the purification of a protein with a cancer metastasis-promoting activity. The monoclonal antibody can be advantageously used as a diagnostic agent for cancer metastases, as well as a cancer metastasis-inhibitory agent. The actual use of the monoclonal antibody as a diagnostic agent are carried out, for example, by collecting a body fluid such as blood, urine, sweat, serum, blood plasma, exudate, and abdominal dropsy from cancer patients, and checking the presence of a cancer metastasis-promoting activity in their body fluids, or by assaying the level of the activity with an immunoassay such as an antigen-antibody reaction, radioimmunoassay (RIA) and enzyme-linked immunosorbent assay (ELISA). Thus, the potential for metastasis of cancer patients can be diagnosed. Furthermore, metastases of cancers in cancer patients can be effectively inhibited by administering the patient with the monoclonal antibody to specifically neutralize a substance with a cancer metastasis-promoting activity.

Thus, the present novel protein has an activity to promote metastasis of cancers. As described above, it has been deemed that metastases of cancers occur or being induced through complicated processes, and because of this the present invention has a great significance in the studies on cancer metastases. The present novel protein and antibody, which recognizes a partial amino acid sequence on the protein, can be advantageously used as a diagnostic agent for cancer metastases, as well as a cancer metastasis-inhibitory agent.

While there has been described what is at present considered to be the preferred embodiments of the invention, it will be understood the various modifications may be made therein, and it is intended to cover in the appended claims all such modifications as fall within the true spirits and scope of the invention.

## Claims

1. A novel protein which has the following physicochemical properties:
(1) Molecular weight
45,000±5,000;
(2) Isoelectric point
pI = 6.0±1.0;
(3) Partial amino acid sequence
Possessing partial amino acid sequences of Glu-Thr-Asp-Lys-Glu-Gly and Phe-Pro-Asn-Asp-Glu-Ala-Lys;
(4) Ultraviolet absorption spectrum
Exhibiting a maximum absorption spectrum around a wave length of 280nm;
(5) Solubility in solvent
Soluble in water, physiological saline and phosphate buffer;
(6) Cancer metastasis-promoting activity
Possessing an activity to promote the metastasis of RPMI 4788 cell (FERM BP-2429) derived from human colon cancer; and
(7) Stability of cancer metastasis-promoting activity
Inactivated in an aqueous solution (pH 7.2) under incubating conditions of 100°C for 10 minutes.
Stable in an aqueous solution (pH 7.2) under storage conditions of 4°C for one month.

2. The novel protein of claim 1, wherein said protein is derived from a microorganism of the genus Mycoplasma.

3. An antibody which recognizes a novel protein having the following physicochemical properties:
(1) Molecular weight
45,000±5,000;
(2) Isoelectric point
pI = 6.0±1.0;
(3) Partial amino acid sequence
Possessing partial amino acid sequences of Glu-Thr-Asp-Lys-Glu-Gly and Phe-Pro-Asn-Asp-Glu-Ala-Lys;
(4) Ultraviolet absorption spectrum
Exhibiting a maximum absorption spectrum around a wave length of 280nm;
(5) Solubility in solvent
Soluble in water, physiological saline and phosphate buffer;
(6) Cancer metastasis-promoting activity
Possessing an activity to promote the metastasis of RPMI 4788 cell (FERM BP-2429) derived from human colon cancer; and
(7) Stability of cancer metastasis-promoting activity
Inactivated in an aqueous solution (pH 7.2) under incubating conditions of 100°C for 10 minutes.
Stable in an aqueous solution (pH 7.2) under storage conditions of 4°C for one month.

4. The antibody of claim 3, wherein the isotype of which is IgG2a.

5. A process for preparing a novel protein, which comprises purifying a crude specimen of a novel protein by using an antibody which specifically binds to said protein, and recovering the resultant purified protein, said novel protein having the following physicochemical properties:
(1) Molecular weight
45,000±5,000;
(2) Isoelectric point
pI = 6.0±1.0;
(3) Partial amino acid sequence
Possessing partial amino acid sequences of Glu-Thr-Asp-Lys-Glu-Gly and Phe-Pro-Asn-Asp-Glu-Ala-Lys;
(4) Ultraviolet absorption spectrum
Exhibiting a maximum absorption spectrum around a wave length of 280nm;
(5) Solubility in solvent
Soluble in water, physiological saline and phosphate buffer;
(6) Cancer metastasis-promoting activity
Possessing an activity to promote the metastasis of RPMI 4788 cell (FERM BP-2429) derived from human colon cancer; and
(7) Stability of cancer metastasis-promoting activity
Inactivated in an aqueous solution (pH 7.2) under incubating conditions of 100°C for 10 minutes.
Stable in an aqueous solution (pH 7.2) under storage conditions of 4°C for one month.

6. The process of claim 5, wherein said antibody is an antibody which recognizes a novel protein having the following physicochemical properties:
(1) Molecular weight
45,000±5,000;
(2) Isoelectric point
pI = 6.0±1.0;
(3) Partial amino acid sequence
Possessing partial amino acid sequences of Glu-Thr-Asp-Lys-Glu-Gly and Phe-Pro-Asn-Asp-Glu-Ala-Lys;
(4) Ultraviolet absorption spectrum
Exhibiting a maximum absorption spectrum around a wave length of 280nm;
(5) Solubility in solvent
Soluble in water, physiological saline and phosphate buffer;
(6) Cancer metastasis-promoting activity
Possessing an activity to promote the metastasis of RPMI 4788 cell (FERM BP-2429) derived from human colon cancer; and
(7) Stability of cancer metastasis-promoting activity
Inactivated in an aqueous solution (pH 7.2) under incubating conditions of 100°C for 10 minutes.
Stable in an aqueous solution (pH 7.2) under storage conditions of 4°C for one month.

7. The antibody of claim 5, wherein the isotype of which is IgG2a.


SEQUENCE LISTING

(1) GENERAL INFORMATION:

(i) APPLICANT:
(A) NAME: KABUSHIKI KAISHA HAYASHIBARA SEIBUTSU KAGAKU
KENKYUJO
(B) STREET: 2-3, 1-CHOME, SHIMOISHII, OKAYAMA-SHI
(C) CITY: OKAYAMA
(E) COUNTRY: JAPAN
(F) POSTAL CODE (ZIP): NONE

(ii) TITLE OF INVENTION: NOVEL PROTEIN, AND ITS ANTIBODY AND
PREPARATION

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93303758.2
(vi) PRIOR APPLICATION DATA:
(A) APPLICATION NUMBER: JP 166659/1992
(B) FILING DATE: 14-MAY-1992

(2) INFORMATION FOR SEQ ID NO: 1:

(i) SEQUENCE CHARACTERISTICS:
(A) LENGTH: 6 amino acids
(B) TYPE: amino acid
(D) TOPOLOGY: unknown

(xi) SEQUENCE DESCRIPTION: SEQ ID NO: 1:

Glu Thr Asp Lys Glu Gly
1               5

(2) INFORMATION FOR SEQ ID NO: 2:

(i) SEQUENCE CHARACTERISTICS:
(A) LENGTH: 7 amino acids
(B) TYPE: amino acid
(D) TOPOLOGY: unknown

(xi) SEQUENCE DESCRIPTION: SEQ ID NO: 2:

Phe Pro Asn Asp Glu Ala Lys
1               5